# EUROPEAN PATENT SPECIFICATION

(11) **EP 4 153 085 B1**
(45) Date of publication and mention of the grant of the patent: **18.02.2026**
(21) Application number: 21727239.2
(22) Date of filing: 13.05.2021
(51) Int. Cl.: A61B 34/20, A61B 18/00, A61B 34/10

(54) **SYSTEM FOR INDEPENDENTLY POSITIONING AN ABLATION TOOL AND IMAGING DEVICE**
SYSTEM ZUR UNABHÄNGIGEN POSITIONIERUNG EINES ABLATIONSWERKZEUGS UND BILDGEBUNGSVORRICHTUNG
SYSTÈME POUR POSITIONNER INDÉPENDAMMENT UN OUTIL D'ABLATION ET UN DISPOSITIF D'IMAGERIE

(30) Priority: 22.05.2020 US 202063028749 P
(43) Date of publication of application: 29.03.2023
(73) Proprietor: Mazor Robotics Ltd., 3079830 Caesarea (IL)
(72) Inventor: WEISS, Noam, 3079830 Caesarea (IL); SHMAYAHU, Yizhaq, 3079830 Caesarea (IL)
(74) Representative: Viering, Jentschura & Partner mbB Patent- und Rechtsanwälte
(86) International application number: PCT/IB2021/054120
(87) International publication number: WO 2021/234520

(56) References cited:
- US-A1- 2020 008 875
- US-B2- 8 267 927
- US-B2- 9 439 627

## Description

### FIELD

The present technology is related generally to ablation of target masses and, more particularly, to ablation of target masses using independently positioned ablation tools and imaging devices.

### BACKGROUND

Ablation procedures are used to treat a variety of conditions and/or tissue including tumor removal and cardiac or brain ablations. Ablation procedures use an ablation tool to apply energy to remove or scar tissue and typically involve applying the energy to a targeted area for a duration of time. The duration of time and applied energy rate may be critical, as an ablation held for too long may result in overheating and overcooling and/or damage to healthy tissues.

In this regard, US 8 267 927 B2 discloses that, in planning an ablation procedure, a planned target volume (PTV) is imported, which is typically selected by a doctor but may be computer-identified, an ablation solution comprising a plurality of ablation volumes is generated or selected using a lookup table, ablations sharing a common axis along a line of insertion are grouped into blocks, wherein alternatively, the PTV is enveloped in a sphere, and a pre-computed ablation solution (e.g., a 6- or 14-sphere solution) is identified to cover the PTV sphere, wherein a mathematical algorithm can be executed to increase an axis through the ablation spheres to generate ellipsoidal ablation volumes that envelop the PTV.

Further, US 9 439 627 B2 discloses an ablation planning and navigation system, including a memory configured to store a plurality of images and a controller configured to render the plurality of images in three dimensions, automatically segment the plurality of images to demarcate a target area, and automatically determine a treatment plan based on the target area, wherein the navigation system includes an ultrasound device having a fiducial pattern that obtains an ultrasound image, an ablation needle having a image capture device that captures an image of the fiducial pattern, and a controller that receives the ultrasound image and the fiducial image, wherein the controller determines a position of the ablation needle based on the fiducial image, and the planning and navigation system also includes a controller that receives data from the planning system and the navigation system and a display configured to display the received information.

Moreover, US 2020/008875 A1 discloses further methods, apparatuses and storage mediums for ablation planning and performance, including imaging, evaluating and diagnosing biological objects, such as, but not limited to, lesions and tumors, wherein such devices, systems, methods and storage mediums may be used for radiotherapy applications, e.g., to determine whether to place seed(s) for radiotherapy, wherein a medial axis or a center line for a predetermined biological object (e.g., a lesion or tumor) is determined/found, one or more target points are picked along the medial axis or center line, and the ablation or radiotherapy zones are defined and optimized, and wherein security checks may be performed in one or more embodiments to ensure proper use of the equipment and patient information.

### SUMMARY

In view of the above, the present invention provides a system for performing at least one ablation according to claim 1. Further advantageous embodiments are described in the dependent claims. The methods described herein do not form part of the invention as such, but support the understanding of the claimed invention.

A system for performing at least one ablation according to the present invention comprises: at least one communication interface for communicating with an imaging device and an ablation tool; a processor; and a memory storing instructions for execution by the processor. The instructions, when executed, cause the processor to: receive image data corresponding to a mass to be ablated, segment a volume of the mass to yield a plurality of sub-volumes, each one of the plurality of sub-volumes corresponding to one of a plurality of ablation steps, identify an ablation center of each sub-volume of the plurality of sub-volumes, calculate, for one ablation step of the plurality of ablation steps, an ablation tool position and an imaging device position, the imaging device position being independent of the ablation tool position, cause the ablation tool to be positioned based on the calculated ablation tool position and the imaging device to be positioned based on the calculated imaging device position, and cause the ablation tool to activate based on the one ablation step.

Any of the aspects herein, wherein the instructions, when executed, may further cause the processor to: identify, based on the corresponding ablation center and mass volume, at least one of an ablation size and shape for at least one of the plurality of ablation steps.

Any of the aspects herein, wherein the instructions, when executed, may further cause the processor to identify an updated ablation center and an updated ablation size and shape for a subsequent ablation step of the plurality of ablation steps after the one ablation step is completed; and calculate, for the subsequent ablation step and after the one ablation step of the plurality of ablation steps is completed, an updated ablation tool position and an updated imaging device position.

Any of the aspects herein, wherein the instructions, when executed, may further cause the processor to: cause, for the subsequent ablation step, the ablation tool to be positioned based on the updated ablation tool position and the imaging device to be positioned based on the updated imaging device position.

Any of the aspects herein, wherein the memory may store additional instructions for execution by the processor that, when executed, further cause the processor to identify an ablation technique for the one ablation step, and further wherein the ablation tool position is based at least in part on the identified ablation technique.

Any of the aspects herein, wherein at least one of the imaging device and the ablation tool may be positioned automatically.

Any of the aspects herein, wherein the imaging device may generate real-time image data during activation of the ablation tool.

Any of the aspects herein, wherein the imaging device may be an ultrasound probe.

Any one of the aspects/features/embodiments in combination with any one or more other aspects/features/embodiments.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a flowchart of a method according to at least one embodiment of the present disclosure;
Fig. 3 is another flowchart of a method according to at least one embodiment of the present disclosure;
Fig. 4A is an image of a mass to be ablated according to at least one embodiment of the present disclosure; and
Fig. 4B is another image of a mass to be ablated according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

In some applications, an ablation progress may be monitored by an imager integrated with the ablation tool and positioned at the same position as the ablation tool. However, in some situations, the ablation may cover an area greater than the imager's range and, as such, the imager may not be able to provide adequate images of ablated tissue beyond a certain distance from the ablation tool and the imager. The ablation may also be monitored and evaluated by images taken after the ablation has been completed. However, in some situations, the images may not be taken until a significant time after the ablation has occurred, in which time, the surrounding tissue may swell and prevent a clear determination of whether the entirety of the target mass was successfully ablated. Inadequate imaging in these and other situations may cause patients to experience damage to healthy tissues or not enough ablation to the target mass, such that the patient may be required to undergo another ablation procedure. Such issues are particularly relevant for masses of irregular or nonsymmetrical shape. Furthermore, some imaging devices may take too long to set up for post-procedure imaging, thereby resulting in images obscured by swollen tissue. Several of the embodiments discussed herein provide improved (e.g., more accurate) imaging during and after an ablation procedure.

Embodiments of the present disclosure provide technical solutions to the problems of: (1) independent positioning of an ablation tool and an imaging device; (2) decreasing overall operating time (and cost), while increasing accuracy of ablations; (3) providing images of the ablation immediately after the ablation procedure has been completed.

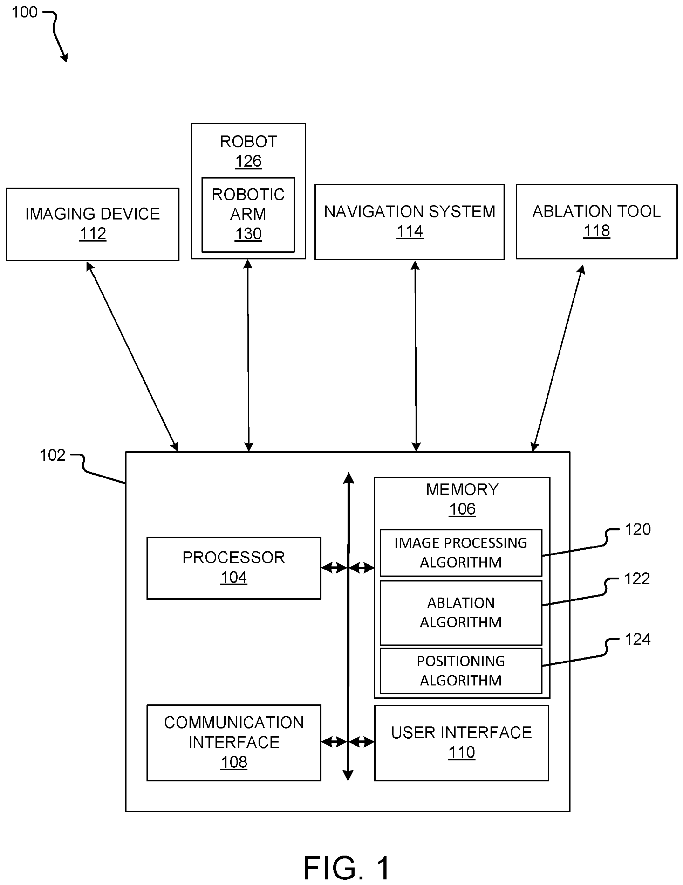

Turning first to Fig. 1, a block diagram of a system 100 according to at least one embodiment of the present disclosure is shown. The system 100 may be used to process image data; execute an ablation algorithm and/or an image processing algorithm; and/or carry out other aspects of one or more of the methods disclosed herein. The system 100 comprises a computing device 102, one or more imaging devices 112, a navigation system 114, a robot 126, and/or one or more ablation tools 118. Systems according to other embodiments of the present disclosure may comprise more or fewer components than the system 100. For example, the system 100 may not include the navigation system 114 and/or the robot 126.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the imaging device 112, the ablation tool 118, the robot 126, and/or the navigation system 114.

The memory 106 may be or comprise RAM, DRAM, SDRAM, other solid-state memory, any memory described herein, or any other tangible, non-transitory memory for storing computer-readable data and/or instructions. The memory 106 may store information or data useful for completing, for example, any step of the methods 200 and 300 described herein. The memory 106 may store, for example, one or more image processing algorithms 120, one or more ablation algorithms 122, and/or one or more positioning algorithms 122. Such instructions or algorithms may, in some embodiments, be organized into one or more applications, modules, packages, layers, or engines. The algorithms and/or instructions may cause the processor 104 to manipulate data stored in the memory 106 and/or received from the imaging device 112 and/or from the ablation tool 118.

The computing device 102 may also comprise a communication interface 108. The communication interface 108 may be used for receiving image data or other information from an external source (such as the imaging device 112, the navigation system 114, the ablation tool 118, and/or the robot 126), and/or for transmitting instructions, images, or other information to an external system or device (e.g., another computing device 102, the navigation system 114, the imaging device 112, the ablation tool 118, and/or the robot 126). The communication interface 108 may comprise one or more wired interfaces (e.g., a USB port, an ethernet port, a Firewire port) and/or one or more wireless interfaces (configured, for example, to transmit information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, touchscreen, and/or any other device for receiving information from a user and/or for providing information to a user. The user interface 110 may be used, for example, to receive a user selection or other user input regarding receiving image data of a mass to be ablated; to receive a user selection or other user input regarding segmenting a volume of the mass; to receive user input regarding identifying an ablation center, an ablation size, and/or an ablation shape; to receive a user selection or other user input regarding calculating an ablation tool position and an imaging device position; to receive a user selection or other user input regarding causing the ablation tool 118 to be positioned based on the calculated ablation tool position and the imaging device 112 to be positioned based on the calculated imaging device position; to receive a user selection or other user input regarding causing the ablation tool 118 to activate based on one of the plurality of ablation steps; to display the image data and/or real-time image data; and/or to display instructions for moving the imaging device 112 and/or the ablation tool 118 or causing the imaging device 112 and/or the ablation tool 118 to move, though it will be appreciated that each of the preceding inputs may be generated automatically by the system 100 (e.g., by the processor 104 or another component of the system 100) or received by the system 100 from a source external to the system 100. In some embodiments, the user interface 110 may be useful to allow a surgeon or other user to modify the instructions or other information displayed.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computer device 102.

The imaging device 112 may be operable to image an object or mass to be ablated (e.g., a tumor) to yield first image data (e.g., image data depicting or corresponding to a tumor). The first image data may be pre-operative image data in some examples, though the first image data may be generated prior to the ablation procedure. The imaging device 112 may be capable of taking a 2D image or a 3D image to yield the image data. "Image data" as used herein refers to the data generated or captured by an imaging device, including in a machine-readable form, a graphical form, and in any other form. In various examples, the image data may comprise data corresponding to a mass of a patient, or to a portion thereof (e.g., the tumor of the patient or a portion thereof). The imaging device 112 may be or comprise, for example, an ultrasound scanner, but may also be or comprise an O-arm, a C-arm, a G-arm, or any other device utilizing X-ray-based imaging (e.g., a fluoroscope, a CT scanner, or other X-ray machine), a magnetic resonance imaging (MRI) scanner, an optical computed tomography scanner, an endoscope, a telescope, a thermographic camera (e.g., an infrared camera), or any other imaging device suitable for obtaining images of a mass of a patient.

The imaging device 112 may additionally or alternatively be operable to image the mass in real-time to yield second image data (e.g., real-time image data and/or intraoperative image data) that may be displayed during an ablation procedure. The second image data may also be combined with the first image data to improve a resolution of the first image data. In some embodiments, the imaging device 112 may comprise more than one imaging device 112. For example, a first imaging device may provide one or more preoperative images of the mass to be ablated and a second imaging device may provide one or more intraoperative images of the mass during the ablation procedure. In other embodiments, the same imaging device may be used to provide both preoperative image(s) and intraoperative image(s).

The navigation system 114 may provide navigation for a surgeon and/or a surgical robot during an operation. The navigation system 114 may be any now-known or future-developed navigation system, including, for example, the Medtronic StealthStation^{™} S8 surgical navigation system. The navigation system 114 may include a camera or other sensor(s) for tracking one or more reference markers, navigated trackers, or other objects within the operating room or other room where an ablation procedure takes place. In various embodiments, the navigation system 114 may be used to track a position of the imaging device 112 (or, more particularly, of a navigated tracker attached, directly or indirectly, in fixed relation to the imaging device 112), and of the ablation tool 118 (or, more particularly, of a navigated tracker attached, directly or indirectly, in fixed relation to the ablation tool 118). The navigation system 114 may include a display for displaying one or more images from an external source (e.g., the computing device 102, imaging device 112, or other source) or a video stream from the camera or other sensor of the navigation system 114. In some embodiments, the system 100 can operate without the use of navigation system 114.

The robot 126 may be any surgical robot or surgical robotic system. The robot 126 may be or comprise, for example, the Mazor X^{™} Stealth Edition robotic guidance system. The robot 126 may comprise one or more robotic arms 130. In some embodiments, the robotic arm 130 may comprise a first robotic arm and a second robotic arm, though the robot 126 may comprise one robotic arm, two robotic arms, or more than two robotic arms. The first robotic arm may hold or otherwise support the ablation tool 118 and the second robotic arm may hold or otherwise support the imaging device 112. The ablation tool 118 and the imaging device 112 may be disposed on an end of the first robotic arm and the second robotic arm, respectively, while in other examples the ablation tool 118 and the imaging device 112 may be disposed on any portion of the first robotic arm, the second robotic arm, and/or the robot 126. In some examples, the imaging device 112 may be an x-ray device having an x-ray source and an x-ray detector, and the robotic arms 130 may comprise a first robotic arm for supporting the ablation tool 118, a second robotic arm for supporting the x-ray source, and a third robotic arm for supporting the x-ray detector.

Reference markers (i.e., navigation markers) may be placed on the robot 126, the robotic arm 130, the imaging device 112, the ablation tool 118, or any other object in the surgical space. The reference markers may be tracked by the navigation system 114, and the results of the tracking may be used by the robot 126 and/or by an operator of the system 100 or any component thereof. In some embodiments, the navigation system 114 can be used to track other components of the system (e.g., imaging device 112 and ablation tool 118) and the system can operate without the use of the robot 126 (e.g., with the surgeon manually manipulating the imaging device 112 and ablation tool 118).

The ablation tool 118 may treat a mass (e.g., a tumor) or neutralize neural activity by applying energy to scar or destroy at least a portion of the mass. The energy may be, but is not limited to, microwave, radiofrequency (RF), cryogenics, ultrasound, laser, or electrophoresis. The ablation tool 118 typically generates a spherical or cylindrical ablation volume, though the ablation tool 118 may generate ablation volumes with other shapes. In other words, a single ablation procedure may result in a substantially spherical void in the mass where the ablation was applied. A size of the spherical ablation volume depends on the duration of the ablation procedure, the type of energy applied, and an amount of energy applied. Some forms of energy may produce a larger ablation volume than other forms of energy. In some examples, one ablation tool 118 is used for the entire ablation procedure. In other examples, two or more ablation tools 118 may be used through the ablation procedure.

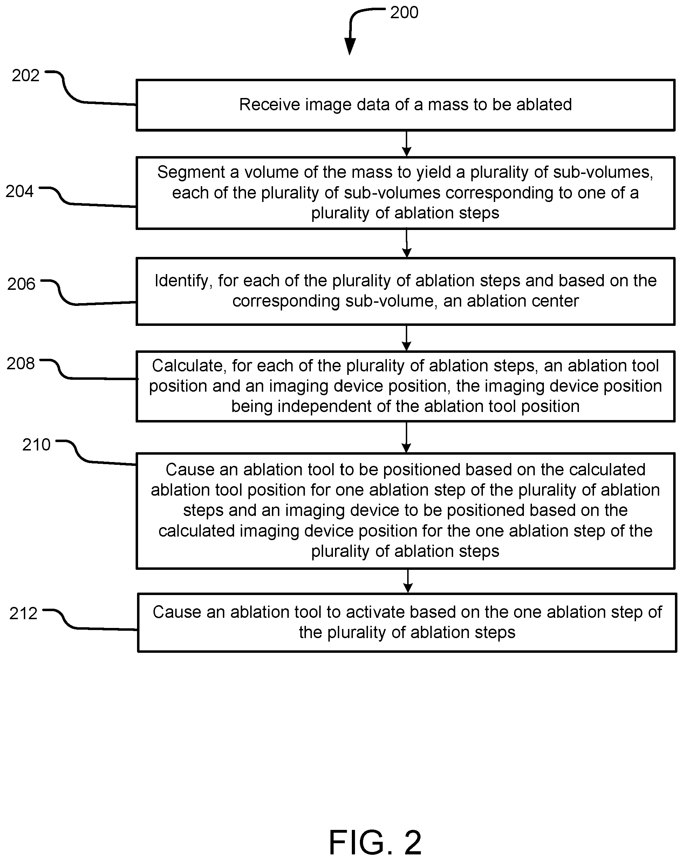

Turning now to Fig. 2, a method 200 for performing at least one ablation according to embodiments of the present disclosure may be executed in whole or in part on a computing device 102. The ablation may be performed by a surgical robot, a surgeon, or a combination of both using one or more ablation tools 118.

The method 200 comprises receiving image data (e.g., first image data) of a mass to be ablated (step 202). The mass to be ablated may be a tumor in some embodiments. In other examples, the mass may be other soft tissue (i.e., heart, uterus, brain, etc.), a vein, skin, or any other anatomical feature of a patient.

The image data may correspond to one or more 2D or 3D preoperative images taken by the imaging device 112. The image data may be generated by the imaging device 112 or by another imaging device, and may be received directly from the imaging device 112 or other imaging device, or indirectly via any other component of the system 100 or network node. The image data may be received via the communication interface 108. The image data may be registered to an ablation coordinate system (e.g. which may be a patient coordinate system), a robotic coordinate system using the robot 126, and/or to a navigation coordinate system using the navigation system 114. In embodiments where the robot 126 holds each of a first imaging device 112 that provides the image data (e.g., first image data), a second imaging device 112 that provides another set of image data (e.g., second image data and/or real-time image data), and the ablation tool 118 (e.g., using multiple robotic arms 130), only a single registration between a robot coordinate system and the ablation/patient coordinate system may be sufficient.

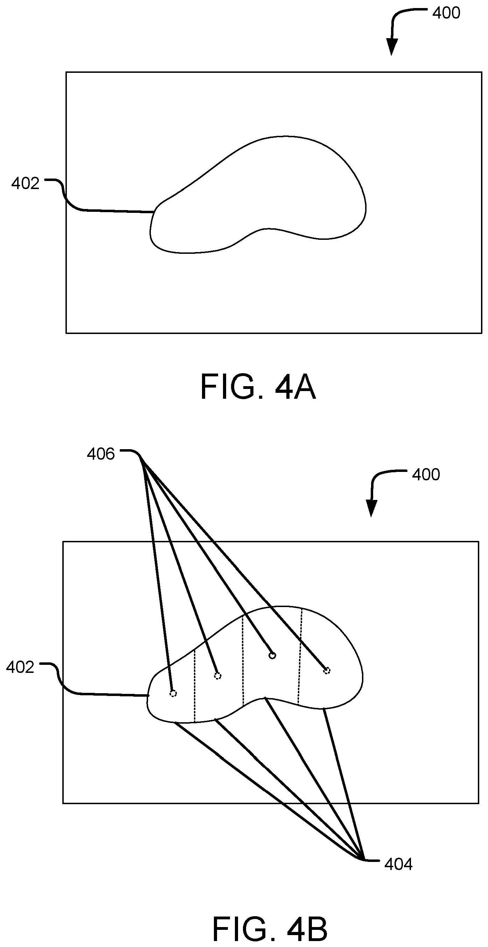

Processing of the image data may include applying the image processing algorithm 120 to the image data (or, alternatively, inputting the image data to the image processing algorithm 120), which algorithm 120 may apply one or more filters to the image data to prepare the image data for further processing. The image data may include or correspond to, for example, the image data 400 shown in Figs. 4A and 4B. As illustrated in Fig. 4A, for example, the image data 400 shows a mass 402 that may be of an irregular shape. In other examples, the mass 402 may be a shape or combination of shapes including, but not limited to, spherical, cylindrical, rectangular, triangular, or oblong.

The method 200 also comprises segmenting, via the image processing algorithm 120, a volume of the mass 402 to yield a plurality of sub-volumes (step 204). The image processing algorithm 120 may be configured to segment the mass 402 volume depicted in the first image data 400, as illustrated in Fig. 4B (in which the center of each sub-volume has been identified). In some embodiments, the image processing algorithm 120 may be configured to evaluate the resulting sub-volumes 404. As illustrated in Fig. 4B, the mass 402 may be segmented by planes to yield the plurality of sub-volumes 404, though in other examples, the mass 402 may be segmented by any shape (including, e.g., spheres or cones). The segmentation shape may be determined, for example, by the type or types of ablation tools 118 to be used to ablate the mass 402. In some examples, each sub-volume of the plurality of sub-volumes 404 may be segmented by a different shape. Though the image data 400 in Fig. 4B appears as a 2D image, it will be appreciated that the image data 400 can be a 3D image or a 2D image, or a combination of 2D images and 3D images. The image data 400 may also be or comprise a 3D model generated by combining a plurality of 2D and/or 3D images.

Each of the plurality of sub-volumes 404 correspond to one of a plurality of ablation steps. Each ablation step includes one or more applications of an ablation tool 118. In some embodiments, each of the plurality of sub-volumes 404 may be ablated during a separate ablation step during the procedure. In other words, a single application of the ablation tool 118 corresponds to one ablation step and ablates each sub-volume. In other embodiments, one or more ablation steps may each include two or more applications of the ablation tool 118, or application of two or more ablation tools 118, whether simultaneously (e.g., as manipulated using multiple robotic arms 130, or by multiple surgeons or other users) or sequentially. Each ablation step may be performed during the ablation procedure using the ablation tool 118. In some embodiments, one ablation tool 118 may be used to perform all of the plurality of ablation steps. In other embodiments, one ablation tool 118 may be used to perform a first one of the plurality of ablation steps, and a second ablation tool 118 may be used to perform a second one of the plurality of ablation steps. In some embodiments, more than two ablation tools 118 may be used for the plurality of ablation steps.

The method 200 also comprises identifying, for each of the plurality of ablation steps and based on the corresponding sub-volume, an ablation center 406 (step 206). The method may also include identifying, for each of the plurality of sub-volumes, an ablation size and/or an ablation shape. The ablation algorithm 122 may be configured to identify the ablation center 406, the ablation size, and/or the ablation shape based on the corresponding sub-volume 404, and/or based on the specific ablation tool 118 to be used to ablate the sub-volume in question. The ablation size and/or the ablation shape may further be based on the ablation center 406 and the corresponding sub-volume.

The ablation shape may correspond to a shape of the ablation, which may be based on the type of ablation tool 118 used for an ablation step and/or an energy selected for an ablation step. For example, some ablation tools may generate a spherical ablation, whereas other ablation tools may ablate a cylindrical volume. The ablation size may correspond to a size of the ablation, such as a diameter, width, height, and/or length. The ablation size may also correspond to a specific ablation shape, such as a diameter of spherical ablation or a diameter and a length of a cylindrical ablation. The ablation size may be based on the energy type and/or intensity selected and/or a duration of the application of the ablation energy. For example, a longer ablation may generate a larger ablation size. The ablation size may also be based on the specific ablation tool selected, as different ablation tools may be better suited for ablating volumes of different sizes.

In some examples, the method 200 may further comprise selecting an ablation technique to perform all or each of the plurality of ablation steps. The ablation technique may include, for example, the specific ablation tool 118 to be used, a frequency and/or amplitude at which ablation energy will be applied during the ablation step, any required movements of the ablation tool 118 during the ablation step, and/or any other settings, configurations, and/or operating information relevant to proper use of the ablation tool 118. In the same examples, the ablation size and/or ablation shape for each of the plurality of ablation steps may be based on the ablation technique selected.

In other examples, the method 200 may further comprise selecting a first ablation technique to perform a first one of the plurality of ablation steps, and selecting a second ablation technique to perform a second one of the plurality of ablation steps. In the same examples, the ablation size and/or ablation shape for the first one of the plurality of ablation steps may be based on the first ablation technique selected and the ablation size and/or ablation shape for the second one of the plurality of ablation steps may be based on the second ablation technique selected. Similarly, a different ablation technique may be selected for different ablation steps, and the corresponding ablation size and/or ablation shape may be based on the selected ablation technique for the corresponding ablation step. In other words, different ablation techniques or energies (e.g., microwave, radiofrequency (RF), cryogenics, ultrasound, laser, or electrophoresis) may be used for different ablation steps. For example, one ablation technique and/or energy may be used for one ablation step to create a larger ablation and another ablation technique and/or energy may be used for another ablation step to create a smaller or finer ablation.

The method 200 also comprises calculating, for each of the plurality of ablation steps, an ablation tool position and an imaging device position (step 208). The positioning algorithm 124 may be configured to calculate the ablation tool position and the imaging device position for each of the ablation steps. The imaging device position is independent of the ablation tool position. The imaging device position and the ablation tool position may include coordinates and/or an orientation of the imaging device 112 and the ablation tool 118, respectively. Calculating the ablation tool position and the imaging device position can be based on the ablation center, ablation size, and/or ablation shape identified in step 206. In some examples, the ablation tool position may be calculated to position an end of the ablation tool 118 at one of the plurality of ablation centers 406 of the corresponding ablation step. In other examples, the ablation tool position can be calculated to position the ablation tool 118 anywhere within or near the mass 402. In some embodiments, the imaging device position is different than the ablation tool position. For example, the imaging device position may be positioned at a distance from the ablation tool position. The position of the imaging device relative to the ablation tool may change for each ablation step, and/or during a single ablation step.

The method 200 further comprises causing the ablation tool 118 to be positioned based on the calculated ablation tool position for one ablation step of the plurality of ablation steps and the imaging device 112 to be positioned based on the calculated imaging device position for the one ablation step of the plurality of ablation steps (step 210). The imaging device 112 may be positioned internal and/or external to the patient. As previously described, internal imaging devices 112 may include, but are not limited to, an endoscope, a probe, or a telescope, and external imaging devices 112 may include, but are not limited to, an ultrasound probe. In some embodiments, one imaging device 112 may be used during the ablation procedure to provide intraoperative image(s). In other embodiments, two or more imaging devices 112 may be used during the ablation procedure to provide the intraoperative image(s). When two or more imaging devices 112 are used, a first one or more of the imaging devices 112 may be used internally and a second one or more of the imaging devices 112 may be used externally, though in other examples each of the two or more imaging devices 112 may be used internally or externally.

The ablation tool 118 and the imaging device 112 may be positioned by the robotic arm 130. For example, a first robotic arm 130 may position the ablation tool 118 and a second robotic arm 130 may independently position the imaging device 112. Prior to positioning the ablation tool 118 and/or the imaging device 112, the robot 126 may perform an incision and/or form one or more paths to the mass 402 or to an area near the mass 402 for the ablation tool 118 and/or the imaging device 112. In other embodiments, a surgeon may perform such an incision and/or form one or more paths to the mass 402 or to an area near the mass 402 for the ablation tool 118 and/or the imaging device 112.

The imaging device 112 may be positioned near the ablation tool 118 or at a distance from the ablation tool 118. The ablation tool 118 and the imaging device 112 may be positioned automatically by the robotic arm(s) 130 in some embodiments. In other embodiments, one or both of the ablation tool 118 or the imaging device 112 may be positioned manually. For example, the ablation tool 118 may be positioned automatically by the robotic arm 130 and the imaging device 112 may be held by a surgeon or other user or held by another robotic arm that can be manually positioned by the surgeon or other user. In other embodiments, the robotic arm 130 may include one or more motors and/or one or more controllers configured to automatically move the imaging device 112 and/or the ablation tool 118 into position.

The method 200 also comprises causing the ablation tool 118 to activate based the one ablation step of the plurality of ablation steps (step 212). Activation of the ablation tool 118 may correspond or be conducted in accordance with a selected ablation technique for the ablation step. The ablation tool 118, the imaging device 112 may provide real-time image data (e.g., second image data) of the ablation at least while the ablation tool 118 is activated. In other words, the real-time image data may be displaying before, during, or after the ablation tool 118 is activated. The real-time image data may be displayed, for example, on the user interface 110, so that the surgeon or other operator may monitor the progress of the ablation procedure in real time.

Further, the method 200 may include causing the imaging device 112 to be automatically or manually repositioned during activation of the ablation tool 118. In other words, the imaging device 112 may be moved during activation of the ablation tool 118 to monitor progression of the ablation and the corresponding mass 402. For example, real-time images of the ablation may be monitored and the ablation may be prematurely stopped when potential damage to living tissue is detected. Use of a system 100 as described herein during an ablation is particularly useful for a mass 402 of irregular or highly nonsymmetrical shape because the imaging device 112 can be freely moved to follow the ablation as the ablation travels through the mass 402. Further, the imaging device 112 can be spaced away from the ablation center 406 and/or the ablation tool 118 to view portions of the ablation at the outskirts of the ablation volume, rather than only directly in front of or adjacent to the ablation tool 118. This is particularly beneficial for ablations where the ablation may extend a distance away from the ablation tool 118 and thus, the imaging device 112 can be moved accordingly to follow the ablation.

In other examples, the imaging device 112 may be positioned to monitor a critical mass (e.g., an organ) that the surgeon wishes to avoid during the ablation procedure. Further, in some embodiments, the imaging device 112 may be automatically moved to a boundary of the critical mass in preparation for or during the ablation procedure. In other embodiments, the imaging device 112 may be automatically moved to a boundary of the mass 402 (e.g., the tumor or other mass to be ablated) in preparation for or during the ablation procedure. The imaging device 112 may also be automatically and continuously moved to a boundary of the ablation during the ablation procedure.

The imaging device 112 may also be moved after the ablation is completed to view and/or analyze results of one or all of the plurality of ablation steps. Immediate high-resolution imaging of the mass 402 (using the imaging device 112) after the ablation is beneficial because tissue may begin to swell minutes after completion of the ablation, thereby obstructing the view of the remaining tissue. Thus, viewing of the mass 402 immediately after the ablation allows for an improved analysis of the remaining mass 402 and ablation effectiveness. As such, movement of the imaging device 112 during or after activation of the ablation tool 118 is highly beneficial to verify that the ablation procedure achieved a correct ablation size and/or ablation shape, to view if surrounding tissues were affected, and/or to determine if the ablation procedure needs adjustments or changes for remaining ablation steps.

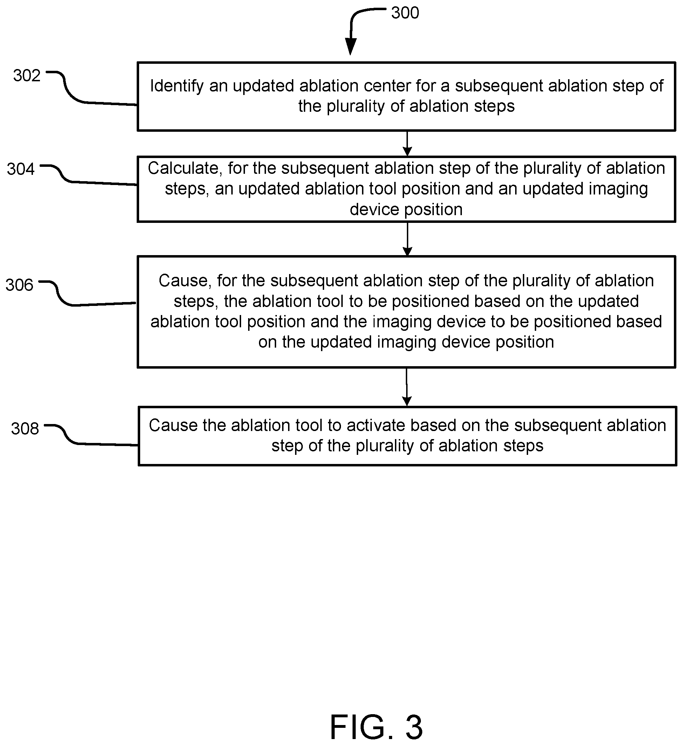

After one ablation step of the plurality of ablation steps are executed, one or more of steps 204-212 may be repeated until all sub-volumes 404 have been ablated. After all of the plurality of ablation steps are executed, the mass 402 may be analyzed for any remaining mass 402 to be ablated. If a further mass 402 requires ablation after one or all of the plurality of ablation steps are executed, steps 206 to 212 may be repeated, as described below with respect to Fig. 3. Such repetition of the steps may provide real-time and continuous calculations of the plurality of ablation steps based on real-time feedback, thereby improving the accuracy of the ablation procedure. Such improved accuracy may result in reduced tissue damage typically experienced during conventional ablation procedures.

Turning now to Fig. 3, a method 300 for updating a plurality of ablation steps according to embodiments of the present disclosure may be executed in whole or in part on a computing device 102. The method 300 may be carried out or otherwise occur when the plurality of ablation steps require updating after one or all of the plurality of ablation steps have occurred.

The method 300 comprises identifying an updated ablation center for a subsequent ablation step of the plurality of ablation steps (step 302). The method 300 may also comprise identifying an updated ablation size and an updated ablation shape. Similar to step 206 as described above, the ablation algorithm 122 may be configured to identify the updated ablation center 406, the updated ablation size, and/or the updated ablation shape for one or more remaining sub-volumes 404. The updated ablation size and the updated ablation shape may further be based on the updated ablation center 406 and the corresponding sub-volume 404.

In some examples, the method 300 may further comprise selecting an updated ablation technique for use in performing the subsequent one of the plurality of ablation steps. In the same examples, the updated ablation size and updated ablation shape for each of the plurality of ablation steps may be based on the updated ablation technique selected. In other examples, the method 300 may further comprise selecting a first updated ablation technique to perform a first subsequent one of the plurality of ablation steps, and selecting a second updated ablation technique to perform a second subsequent one of the plurality of ablation steps. In the same examples, the updated ablation size and updated ablation shape for the first subsequent one of the plurality of ablation steps may be based on the first updated ablation technique selected and the updated ablation size and updated ablation shape for the second subsequent one of the plurality of ablation steps may be based on the second updated ablation technique selected. Similarly, a different updated ablation technique may be selected for different ablation steps, and the corresponding updated ablation size and updated ablation shape may be based on the selected updated ablation technique for the corresponding ablation step.

The method 300 also comprises calculating, for the subsequent ablation step of the plurality of ablation steps, an updated ablation tool position and an updated imaging device position (step 304). Similar to step 208 as described above, the updated imaging device position and the updated ablation tool position may include coordinates and/or an orientation of the imaging device 112 and the ablation tool 118, respectively. Calculating the updated ablation tool position and the updated imaging device position can be based on the updated ablation size and updated ablation shape identified in step 302. In some examples, the updated ablation tool position may be calculated to position an end of the ablation tool 118 at one of the plurality of updated ablation centers 406 of the corresponding ablation step. In other examples, the updated ablation tool position can be calculated to position the ablation tool 118 anywhere within or near the mass 402. The updated imaging device position may also be calculated to position the imaging device at a distance from the ablation tool.

The method 300 further comprises causing, for the subsequent ablation step of the plurality of ablation steps, the ablation tool to be positioned based on the updated ablation tool position and the imaging device to be positioned based on the updated imaging device position (step 306). Similar to step 210 as described above, the imaging device 112 may be positioned internal and/or external to the patient based on the updated imaging device position.

Each of the ablation tool 118 may be positioned at the updated ablation tool position and the imaging device 112 may be positioned at the updated imaging device position by the robotic arm 130. For example, a first robotic arm may position the ablation tool 118 and a second robotic arm may independently position the imaging device 112. Prior to positioning the ablation tool 118 and/or the imaging device 112, the robot 126 may also perform a subsequent incision and/or form a subsequent one or more paths to the mass 402 or to an area near the mass 402 for the ablation tool 118 and/or the imaging device 112.

The ablation tool 118 and the imaging device 112 may be positioned automatically by the robotic arm 130 in some embodiments (e.g., using either one or multiple robotic arms). In other embodiments, one or both of the ablation tool 118 or the imaging device 112 may be positioned manually. For example, the ablation tool 118 may be positioned automatically by the robotic arm 130 and the imaging device 112 may be held by a surgeon or other operator or held by another arm (robotic or otherwise) that can be manually positioned by the surgeon or other user. In other embodiments, the robotic arm 130 may include one or more motors and/or one or more controllers configured to automatically move the imaging device 112 and/or the ablation tool 118 into position.

The method 300 also comprises causing the ablation tool to activate based on the subsequent ablation step of the plurality of ablation steps (step 308). Similar to step 212 as described above, during activation of the ablation tool 118, the imaging device 112 may provide subsequent real-time image data (e.g., subsequent real-time image data) of the ablation. The subsequent real-time image data may be displayed, for example, on the user interface 110. Further, the method 200 may include causing the imaging device 112 to be automatically or manually repositioned during the activation of the ablation tool 118. In other words, the imaging device 112 may be moved during activation of the ablation tool 118 to monitor progression of the ablation and the corresponding mass 402.

The methods and systems described herein provide improved real-time imaging of an ablation and corresponding mass based on independent positioning of an imaging device and an ablation tool, thereby allowing for analysis of the ablation and/or updates to the ablation procedure in real-time. The methods and systems also provide for improved post-ablation imaging of the mass, thereby allowed for a more accurate analysis of the effectiveness of the individual ablation.

As may be appreciated based on the foregoing disclosure, the present disclosure encompasses methods with fewer than all of the steps identified in Figs. 2 and 3 (and the corresponding description of the methods 200 and 300), as well as methods that include additional steps beyond those identified in Figs. 2 and 3 (and the corresponding description of the methods 200 and 300).

The foregoing discussion has been presented for purposes of illustration and description. The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the appended claims.

## Claims

1. A system (100) for performing at least one ablation comprising:
at least one communication interface (108) for communicating with an imaging device (112) and an ablation tool (118);
a processor (104); and
a memory (106) storing instructions for execution by the processor (104) that, when executed, cause the processor (104) to:
receive image data corresponding to a mass to be ablated;
segment a volume of the mass to yield a plurality of sub-volumes, each one of the plurality of sub-volumes corresponding to one of a plurality of ablation steps;
identify an ablation center of each sub-volume of the plurality of sub-volumes;
calculate, for one ablation step of the plurality of ablation steps, an ablation tool position and an imaging device position, the imaging device position being independent of the ablation tool position,
cause the ablation tool (118) to be positioned based on the calculated ablation tool position and the imaging device (112) to be positioned based on the calculated imaging device position, and
cause the ablation tool (118) to activate based on the one ablation step.

2. The system (100) of claim 1, wherein the instructions, when executed, further cause the processor (104) to:
identify, based on the corresponding ablation center and mass volume, at least one of an ablation size and shape for at least one of the plurality of ablation steps.

3. The system (100) of claim 1, wherein the instructions, when executed, further cause the processor (104) to:
identify an updated ablation center for a subsequent ablation step of the plurality of ablation steps after the one ablation step is completed; and
calculate, for the subsequent ablation step and after the one ablation step of the plurality of ablation steps is completed, an updated ablation tool position and an updated imaging device position.

4. The system (100) of claim 3, wherein the instructions, when executed, further cause the processor (104) to:
cause, for the subsequent ablation step, the ablation tool (118) to be positioned based on the updated ablation tool position and the imaging device (112) to be positioned based on the updated imaging device position.

5. The system (100) of claim 1, wherein at least one of the imaging device (112) and the ablation tool (118) is positioned automatically.

6. The system (100) of claim 1, wherein the imaging device (112) generates real-time image data during activation of the ablation tool (118).

7. The system of claim 1, wherein the imaging device (112) is an ultrasound probe.

8. The system of claim 1, wherein the memory (106) stores additional instructions for execution by the processor (104) that, when executed, further cause the processor (104) to identify an ablation technique for the one ablation step, and further wherein the ablation tool position is based at least in part on the identified ablation technique.

## Patentansprüche

1. System (100) zur Durchführung mindestens einer Ablation, umfassend:
mindestens eine Kommunikationsschnittstelle (108) zur Kommunikation mit einer Bildgebungsvorrichtung (112) und einem Ablationswerkzeug (118);
einen Prozessor (104); und
einen Speicher (106), der Anweisungen für eine Ausführung durch den Prozessor (104) speichert, die, wenn sie ausgeführt werden, den Prozessor (104) veranlassen zum:
Empfangen von Bilddaten entsprechend einer zu abladierenden Masse;
Segmentieren eines Volumens der Masse, um eine Vielzahl von Teilvolumina zu erhalten, wobei jedes der Vielzahl von Teilvolumina einem von einer Vielzahl von Ablationsschritten entspricht;
Identifizieren eines Ablationszentrums jedes Teilvolumens der Vielzahl von Teilvolumina;
Berechnen, für einen Ablationsschritt der Vielzahl von Ablationsschritten, eine Ablationswerkzeugposition und eine Bildgebungsvorrichtungsposition, wobei die Bildgebungsvorrichtungsposition unabhängig von der Ablationswerkzeugposition ist,
Veranlassen, dass das Ablationswerkzeug (118) basierend auf der berechneten Ablationswerkzeugposition positioniert wird und die Bildgebungsvorrichtung (112) basierend auf der berechneten Bildgebungsvorrichtungsposition positioniert wird, und
Veranlassen, dass das Ablationswerkzeug (118) basierend auf dem einen Ablationsschritt aktiviert wird.

2. System (100) nach Anspruch 1, wobei die Anweisungen, wenn sie ausgeführt werden, den Prozessor (104) ferner veranlassen zum:
Identifizieren, basierend auf dem entsprechenden Ablationszentrum und Massenvolumen, von mindestens einem von einer Ablationsgröße und -form für mindestens einen der Vielzahl von Ablationsschritten.

3. System (100) nach Anspruch 1, wobei die Anweisungen, wenn sie ausgeführt werden, den Prozessor (104) ferner veranlassen zum:
Identifizieren eines aktualisierten Ablationszentrums für einen nachfolgenden Ablationsschritt der Vielzahl von Ablationsschritten, nachdem der eine Ablationsschritt abgeschlossen ist; und
Berechnen, für den nachfolgenden Ablationsschritt und nachdem der eine Ablationsschritt der Vielzahl von Ablationsschritten abgeschlossen ist, einer aktualisierten Ablationswerkzeugposition und einer aktualisierten Bildgebungsvorrichtungsposition.

4. System (100) nach Anspruch 3, wobei die Anweisungen, wenn sie ausgeführt werden, den Prozessor (104) ferner veranlassen zum:
Veranlassen, für den nachfolgenden Ablationsschritt, dass das Ablationswerkzeug (118) basierend auf der aktualisierten Ablationswerkzeugposition positioniert wird und die Bildgebungsvorrichtung (112) basierend auf der aktualisierten Bildgebungsvorrichtungsposition positioniert wird.

5. System (100) nach Anspruch 1, wobei mindestens eines von der Bildgebungsvorrichtung (112) und dem Ablationswerkzeug (118) automatisch positioniert wird.

6. System (100) nach Anspruch 1, wobei die Bildgebungsvorrichtung (112) Echtzeit-Bilddaten während der Aktivierung des Ablationswerkzeugs (118) erzeugt.

7. System nach Anspruch 1, wobei die Bildgebungsvorrichtung (112) eine Ultraschallsonde ist.

8. System nach Anspruch 1, wobei der Speicher (106) zusätzliche Anweisungen zur Ausführung durch den Prozessor (104) speichert, die, wenn sie ausgeführt werden, ferner veranlassen, dass der Prozessor (104) eine Ablationstechnik für den einen Ablationsschritt identifiziert, und ferner wobei die Ablationswerkzeugposition mindestens teilweise auf der identifizierten Ablationstechnik basiert.

## Revendications

1. Système (100) permettant d'effectuer au moins une ablation, comprenant :
au moins une interface de communication (108) pour communiquer avec un dispositif d'imagerie (112) et un outil d'ablation (118) ;
un processeur (104) ; et
une mémoire (106) stockant des instructions destinées à être exécutées par le processeur (104) qui, lorsqu'elles sont exécutées, amènent le processeur (104) à :
recevoir des données d'image correspondant à une masse à ablater ;
segmenter un volume de la masse pour obtenir une pluralité de sous-volumes, chacun de la pluralité de sous-volumes correspondant à l'une d'une pluralité d'étapes d'ablation ;
identifier un centre d'ablation de chaque sous-volume de la pluralité de sous-volumes ;
calculer, pour une étape d'ablation de la pluralité d'étapes d'ablation, une position d'outil d'ablation et une position de dispositif d'imagerie, la position de dispositif d'imagerie étant indépendante de la position d'outil d'ablation,
amener l'outil d'ablation (118) à être positionné sur la base de la position calculée d'outil d'ablation et le dispositif d'imagerie (112) à être positionné sur la base de la position calculée de dispositif d'imagerie, et
amener l'outil d'ablation (118) à s'activer sur la base de l'étape d'ablation.

2. Système (100) selon la revendication 1, dans lequel les instructions, lorsqu'elles sont exécutées, amènent en outre le processeur (104) à :
identifier, sur la base du centre d'ablation et du volume de masse correspondants, au moins l'une parmi une taille et une forme d'ablation pour au moins une de la pluralité d'étapes d'ablation.

3. Système (100) selon la revendication 1, dans lequel les instructions, lorsqu'elles sont exécutées, amènent en outre le processeur (104) à :
identifier un centre d'ablation mis à jour pour une étape d'ablation suivante de la pluralité d'étapes d'ablation après l'achèvement de l'étape d'ablation ; et
calculer, pour l'étape d'ablation suivante et après l'achèvement de l'étape d'ablation de la pluralité d'étapes d'ablation, une position d'outil d'ablation mise à jour et une position de dispositif d'imagerie mise à jour.

4. Système (100) selon la revendication 3, dans lequel les instructions, lorsqu'elles sont exécutées, amènent en outre le processeur (104) à :
amener, pour l'étape d'ablation suivante, l'outil d'ablation (118) à être positionné sur la base de la position d'outil d'ablation mise à jour et le dispositif d'imagerie (112) à être positionné sur la base de la position de dispositif d'imagerie mise à jour.

5. Système (100) selon la revendication 1, dans lequel au moins l'un parmi le dispositif d'imagerie (112) et l'outil d'ablation (118) est positionné automatiquement.

6. Système (100) selon la revendication 1, dans lequel le dispositif d'imagerie (112) génère des données d'image en temps réel pendant l'activation de l'outil d'ablation (118).

7. Système selon la revendication 1, dans lequel le dispositif d'imagerie (112) est une sonde à ultrasons.

8. Système selon la revendication 1, dans lequel la mémoire (106) stocke des instructions supplémentaires destinées à être exécutées par le processeur (104) qui, lorsqu'elles sont exécutées, amènent en outre le processeur (104) à identifier une technique d'ablation pour l'étape d'ablation, et en outre dans lequel la position de l'outil d'ablation est basée au moins en partie sur la technique d'ablation identifiée.
